## Europäisches Patentamt
## European Patent Office
## Office européen des brevets

(19)

(11) Veröffentlichungsnummer: **0 350 515**

**A1**

(12) # EUROPÄISCHE PATENTANMELDUNG

(21) Anmeldenummer: **88111044.9**

(22) Anmeldetag: **11.07.88**

(51) Int. Cl.4: **A61L 2/06 , F26B 5/06**

(43) Veröffentlichungstag der Anmeldung:
**17.01.90 Patentblatt 90/03**

(84) Benannte Vertragsstaaten:
**AT BE CH DE ES FR GB IT LI NL SE**

(71) Anmelder: **LEYBOLD AKTIENGESELLSCHAFT**
**Bonner Strasse 498**
**D-5000 Köln 51(DE)**

(72) Erfinder: **May, Heinz**
**Am Römerpfad 32**
**D-5024 Pulheim(DE)**
Erfinder: **Diesing, Hartmuth**
**Akazienweg 5**
**D-5632 Wermelskirchen(DE)**
Erfinder: **Steinkamp, Heinrich**
**Im Garten 7**
**D-5000 Köln 50(DE)**

(74) Vertreter: **Leineweber, Jürgen, Dipl.-Phys.**
**Nagelschmiedshütte 8**
**D-5000 Köln 40(DE)**

(54) **Verfahren zum Betrieb einer industriellen Anlage mit mindestens einer Vakuumkammer und Anlage zur Durchführung dieses Verfahrens.**

(57) Die Erfindung bezieht sich auf ein Verfahren zum Betrieb einer industriellen Anlage (1) mit mindestens einer Vakuumkammer (2, 4), welche vor der Inbetriebnahme sterilisiert wird und nach Beendigung des in der Vakuumkammer durchgeführten Verfahrens mit sterilem Gas belüftet wird, indem das der Belüftung dienende Gas durch ein Sterilfilter (7) in die Vakuumkammer eingelassen wird; um die Gefahr der Belüftung der Kammer mit unsterilem Gas zu reduzieren, wird vorgeschlagen, daß unmittelbar nach der gleichzeitig durchgeführten Sterilisation von Kammer (2, 4) und Filter (7) die Anlage (1) in Betrieb genommen und der in der Kammer durchzuführende Prozeß eingeleitet wird, daß der Filter (7) nach seiner Sterilisation in eingebautem Zustand auf Integrität geprüft wird und daß für den Fall, daß der Filter (7) defekt ist, das Filtermaterial (12) ausgetauscht und der Filter separat von der Vakuumkammer (2, 4) erneut sterilisiert wird.

EP 0 350 515 A1

## Verfahren zum Betrieb einer industriellen Anlage mit mindestens einer Vakuumkammer und Anlage zur Durchführung dieses Verfahrens

Die Erfindung bezieht sich auf ein Verfahren zum Betrieb einer industriellen Anlage mit mindestens einer Vakuumkammer, welche vor der Inbetriebnahme sterilisiert wird und nach Beendigung des in der Vakuumkammer durchgeführten Verfahrens mit sterilem Gas belüftet wird, indem das der Belüftung dienende Gas durch ein Sterilfilter in die Vakuumkammer eingelassen wird. Außerdem bezieht sich die vorliegende Erfindung auf eine für die Durchführung dieses Verfahrens geeignet ausgerüstete Anlage.

Ein Beispiel für eine Anlage der betroffenen Art ist eine Gefriertrocknungsanlage für pharmazeutische Produkte. Diese umfaßt mindestens eine Vakuumkammer und häufig zwei Kondensatoren, die vor ihrer Inbetriebnahme sterilisiert werden müssen. Die Sterilisation erfolgt z. B. in an sich bekannter Weise durch Einlassen von Heißdampf, so daß durch thermische Einwirkung die Innenflächen der Kammern und benachbarter Anlagenteile (Leitungen, Armaturen und dergleichen) von schädlichen Keimen befreit werden.

Ist der in der Gefriertrocknungsanlage durchgeführte Prozeß abgeschlossen, erfolgt eine Belüftung (teilweise oder bis auf Atmosphärendruck) der Kammern. Um das Produkt nicht zu kontaminieren und/oder um die Anlage für die nächste Charge keimfrei zu halten, ist es bekannt, das der Belüftung dienende Gas (Luft, Stickstoff oder dergleichen) über ein Sterilfilter zuzuführen. Im Filtermaterial dieses Filters werden schädliche Keime zurückgehalten. Nur keimfreies Gas gelangt in die Kammern der Anlage.

Auch bei Autoklaven oder ähnlichen, in der Elektronik- und Lebensmittelindustrie eingesetzten Anlagen ist diese Verfahrensweise bekannt.

Notwendige Voraussetzung für die Zuführung von keimfreien Belüftungsgasen ist die fehlerfreie Funktion des Sterilfilters. Es ist deshalb bekannt, vor der Inbetriebnahme einer Gefriertrocknungsanlage, und zwar noch vor ihrer Sterilisation, den Sterilfilter einem sogenannten Integritätstest zu unterwerfen. Dieses geschieht dadurch, daß der Filter oder die Filterkerze bzw. Filterscheibe ausgebaut wird und in einer separaten Meßeinrichtung auf Fehlerfreiheit überprüft wird. Der Test erfolgt z. B. in der Weise, daß die Filterkerze oder die -scheibe benetzt wird und anschließend für eine vorgegebene Zeit einer Druckdifferenz ausgesetzt wird. Erweist sich das Filtermaterial in diesem Druckhaltetest als fehlerfrei, dann erfolgt der Wiedereinbau des Filters.

Nachteilig an dem beschriebenen Verfahren ist, daß Defekte am Filter, die infolge der relativ hohen thermischen Belastung während der Sterilisation entstehen oder deren Ursache in einem fehlerhaften Wiedereinbau des Filters liegen, nicht mehr feststellbar sind. Die Gefahr, daß die Anlage mit unsterilem Gas belüftet wird, ist deshalb nicht ausgeschlossen.

Der vorliegenden Erfindung liegt die Aufgabe zugrunde, ein Verfahren der eingangs genannten Art sowie eine für die Durchführung des Verfahrens geeignet ausgerüstete Anlage vorzuschlagen, bei der die Gefahr der Belüftung der Anlagen-Kammern mit unsterilem Gas nicht mehr besteht.

Erfindungsgemäß wird diese Aufgabe dadurch gelöst, daß unmittelbar nach der gleichzeitig durchgeführten Sterilisation von Kammer und Filter die Anlage in Betrieb genommen und der in der Kammer durchzuführende Prozeß eingeleitet wird, daß der Filter nach seiner Sterilisation in eingebautem Zustand auf Integrität geprüft wird und daß für den Fall, daß der Filter defekt ist, das Filtermaterial ausgetauscht und der Filter separat von der Vakuumkammer erneut sterilisiert wird. Ergibt sich bei der im eingebauten Zustand des Sterilfilters durchgeführten Integritätsprüfung eine fehlerfreie Funktion, dann sind Manipulationen im Bereich des Filters nicht mehr erforderlich. Die Gefahr, daß thermische Schäden durch den Sterilisationsschritt oder Fehlfunktionen infolge eines falschen Wiedereinbaus auftreten, welche vor der Belüftung der Vakuumkammern über den Sterilfilter nicht mehr festgestellt werden, besteht nicht mehr. Ergibt der Integritätstest einen Fehler am Sterilfilter, dann kann dieser Fehler z. B. durch Austausch der Filterkerze beseitigt werden, ohne daß mit der eigentlichen Inbetriebnahme der Anlage gewartet werden muß. Nach dem Austausch der Filterkerze kann der Sterilfilter separat sterilisiert und danach einem Integritätstest unterworfen werden. Die Inbetriebnahme der Anlage wird bei einem Defekt am Sterilfilter nicht mehr verzögert; dadurch, daß es möglich ist, diesen Filter separat zu sterilisieren und den Integritätstest im eingebauten Zustand durchzuführen, kann die Gefahr der Verwendung fehlerhafter Sterilfilter vollständig ausgeschlossen werden. Ein weiterer Vorteil besteht darin, daß der Ablauf des Integritätstestes automatisierbar ist und daß Ablauf, Überwachung und Dokumentierung softwaremäßig in die ohnehin vorhandene Prozeß-und Anlagensteuerung integrierbar ist.

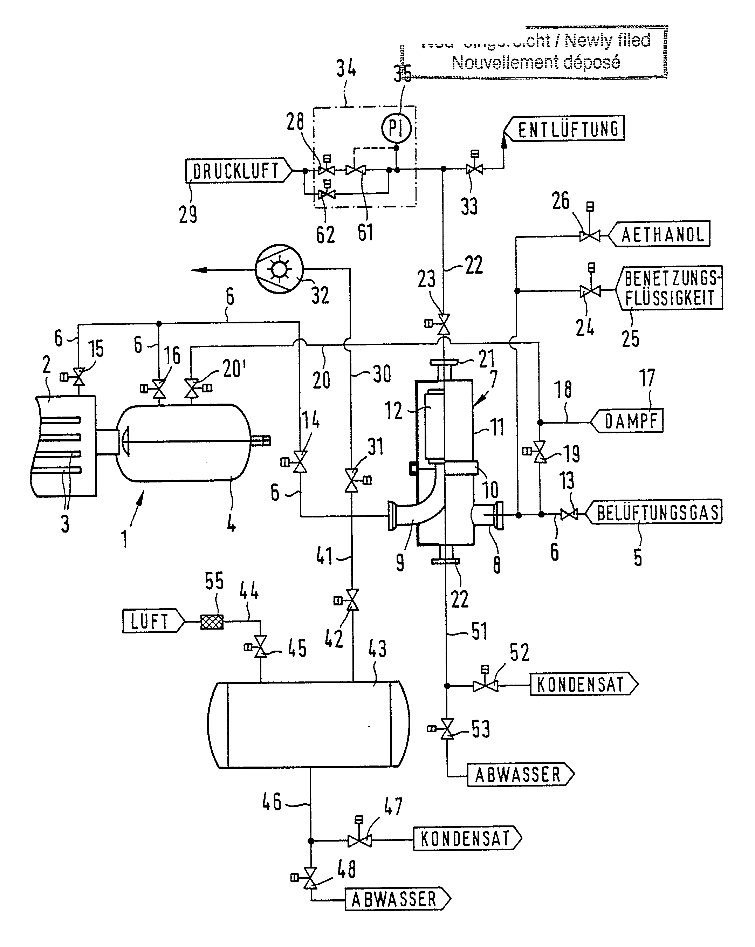

Weitere Vorteile und Einzelheiten der Erfindung sollen am Beispiel einer in der Figur schematisch dargestellten Gefriertrocknungsanlage erläutert werden.

Die Gefriertrocknungsanlage 1 umfaßt die nur teilweise dargestellte Gefriertrocknungskammer 2 mit ihren Stellflächen 3 und den Kondensator 4. Nur für die vorliegende Erfindung relevante Anschlüsse an der Gefriertrocknungskammer 2 und am Kondensator 4 sind dargestellt. Auf die Darstellung weiterer Versorgungsanschlüsse, z. B. für den Kälteerzeuger, die Vakuumpumpe und dergleichen wurde aus Übersichtlichkeitsgründen verzichtet. Eine von einer Belüftungsgasquelle 5 zur Gefriertrocknungskammer 2 und zum Kondensator 4 führende Leitung ist mit 6 bezeichnet. In diese Leitung ist der Sterilfilter 7 mit seinen Anschlüssen 8 und 9 eingebaut. Der teilweise offen dargestellte Sterilfilter 7 umfaßt das Gehäuse 11 und die darin eingebaute zylindrische Filterkerze 12. Zum Zwecke des Austausches der Filterkerze 12 kann das Gehäuse 11 mit Hilfe der Klammer 10 geöffnet werden. Das Innere des Gehäuses 11 ist mit dem Anschluß 8 verbunden, während der Innenraum der Filterkerze 12 mit dem Anschluß 9 in Verbindung steht. Für den Fall, daß die Gefriertrocknungskammer 2 und der Kondensator 4 belüftet werden sollen, durchsetzt das Belüftungsgas die Filterkerze 12 von außen nach innen. In der Leitung 6 befinden sich noch die Ventile 13 (zwischen Belüftungsgasquelle und Anschluß 8 des Sterilfilters 7) das Ventil 14 (in Strömungsrichtung des Belüftungsgases hinter dem Sterilfilter liegend) und die Ventile 15, 16, die in einer Verzweigung der Leitung 6 in unmittelbarer Nähe der Gefriertrocknungskammer 2 bzw. des Kondensators 4 angeordnet sind.

Die Sterilisation der Kammern 2 und 4 der Gefriertrocknungsanlage 1 erfolgt in bekannter Weise mit Hilfe von Heißdampf. Damit gleichzeitig auch der Sterilfilter und die Leitung 6 keimfrei werden, erfolgt die Zuführung des Heißdampfes von der Heißdampfquelle 17 über die Leitung 18 mit dem Ventil 19 in die Leitung 6, und zwar zwischen Ventil 13 und Anschluß 8 des Sterilfilters 7. Falls die über den Sterilfilter 7 strömende Dampfmenge nicht ausreicht, die relativ großen Kammern 2 und 4 des Sterilfilters mit Dampf zu versorgen, kann diesen Kammern zusätzlich Dampf zugeführt werden. Als Beispiel ist die Leitung 20 mit dem Ventil 20′ eingezeichnet, die direkt zum Kondensator 4 führt.

Um den Sterilfilter 7 bzw. seine Filterkerze 12 im eingebauten Zustand auf Integrität prüfen zu können, ist das Gehäuse 11 mit zwei weiteren Anschlüssen 21 und 22 ausgerüstet, von denen einer (21) oberhalb der Filterkerze 12 angeordnet ist, während sich der andere (22) im Bodenbereich des Filtergehäuses 11 befindet. Mit dem Anschluß 21 ist die Leitung 22 mit dem Ventil 23 verbunden. An diese Leitung 22 ist über in parallelen Leitungsabschnitten befindlichen Ventilen 28, 61 bzw. 62 eine Druckluftquelle 29 angeschlossen. Außerdem kann die Leitung 22 über das Ventil 33 entlüftet werden.

Den Ventilen 28, 61, 62 ist eine generell als Block 34 dargestellte Regeleinrichtung zugeordnet, die es erlaubt, die Leitung 22 mit einem bestimmten Druck zur Durchführung eines Druckhaltetestes zu versorgen. Die Regeleinrichtung 34 ist mit einer Vorrichtung 35 zur Beobachtung des Druckes in der Leitung 22 nach dem Schließen der Ventile 28, 62 versehen. Zweckmäßig sind noch im einzelnen nicht dargestellte Protokollierungseinrichtungen vorgesehen, damit eine spätere Dokumentation der Testdurchführung und seiner Ergebnisse möglich ist.

In die Leitung 6 zwischen Ventil 13 und Anschluß 8 mündet eine Leitung 60, an die über das Ventil 24 ein Behälter 25 mit Benetzungsflüssigkeit und über das Ventil 26 ein Behälter mit Alkohol angeschlossen sind.

In die Leitung 6 zwischen Ventil 14 und Anschluß 9 mündet die Leitung 30 mit dem Ventil 31, welche zu einer Vakuumpumpe 32, vorzugsweise einer Wasserringpumpe, führt. In die Leitung 6 zwischen Ventil 14 und Anschluß 9 des Sterilfilters 7 mündet weiterhin die Leitung 41 mit dem Ventil 42, die zu einem Behälter 43 führt. Dieser Behälter ist - wie weiter unten beschrieben -u. a. zur Durchführung des Druckhaltetestes des Sterilfilters 7 im eingebauten Zustand zweckmäßig. Über die Leitung 44 und dem Ventil·45 kann der Behälter belüftet werden. Im Bodenbereich des Behälters 43 ist außerdem noch das Leitungssystem 46 mit den Ventilen 47 und 48 angeschlossen, welches der Abführung von Kondensat und Abwasser aus dem Behälter 43 dient. Auch an den Anschluß 22 des Sterilfilters 7 ist ein Leitungssystem 51 mit den Ventilen 52 und 53 angeschlossen, welches der Abführung von Kondensat und Abwasser aus dem Gehäuse 11 des Sterilfilters dient.

Vor der eigentlichen Inbetriebnahme der Gefriertrocknungsanlage 1 werden die Kammern 2 und 4 und der Sterilfilter 7 sterilisiert, indem Dampf aus der Dampfversorgung 17 über die Leitungen 18, 20 die Leitung 6 und den Sterilfilter 7 den Kammern 2 und 4 zugeführt wird. Die Ventile 14 bis 16, 19 und 20′ sind während der Sterilisationsphase geöffnet, während alle übrigen Ventile geschlossen sind. Für den Fall, daß der Behälter 43 ebenfalls sterilisiert wird, ist auch das Ventil 42 geöffnet. Nach dem Sterilisationsschritt werden die Ventile 14 bis 16, 19, 20′ und gegebenenfalls 42 geschlossen. Mit dem in der Gefriertrocknungsanlage 1 durchzuführenden Prozeß kann unmittelbar danach begonnen werden.

Unabhängig von dem in der Gefriertrocknungsanlage ablaufenden Prozeß, kann nach dem Sterilisationsschritt die Filterkerze 12 auf Integrität untersucht werden. Für diese Untersuchung ist ein sogenannter Druckhaltetest durchzuführen. Der Druckhaltetest beinhaltet folgende Arbeitsschritte:

a) Benetzen der Filterkerze mit einer Netzflüssigkeit

Zu diesem Zweck wird das belüftete Filtergehäuse 11 über das geöffnete Ventil 24 und Leitung 60 mit einer Benetzungsflüssigkeit komplett geflutet. Die Ventile 23, 42, 45 und 33 sind geöffnet.

b) Entleeren der Benetzungsflüssigkeit aus dem Filtergehäuse 11

Die Entleerung erfolgt über die Leitung 51 bei geöffneten Ventilen 53, 23, 33, 42, 45.

c) Aufbau Testdruck

Das Filtergehäuse 11 wird bei geöffnetem Ventil 28, 61 mit Druckluft bis zu einem Testdruck, z. B. ein Bar Überdruck, geflutet.

d) Stabilisieren Testdruck

Der Testdruck wird für einen Zeitraum von z. B. 300 s konstant gehalten. Druckverluste werden in dieser Zeit durch Öffnen des Ventils 28 ausgeglichen.

e) Testen Filterkerze (messen Druckabfall)

Nach einer Zeit von z. B. weiteren 300 s wird der Druckabfall des Testdruckes gemessen. Ist dieser Druckabfall kleiner z. B. 50 mbar, so ist die Filterkerze fehlerfrei.

f) Sammelbehälter "entleeren"

Die im Sammelbehälter 43 angesammelte Benetzungsflüssigkeit wird über Leitung 46 und Ventil 48 entleert.

g) Benetzen der Filterkerze mit Aethanol

Das Filtergehäuse wird über Ventil 26 und Leitung 60 mit Aethanol komplett geflutet. Die Ventile 23, 42, 45 und 33 sind wieder geöffnet.

h) Entleeren des Filtergehäuses

Die Entleerung erfolgt über die Leitung 51 bei geöffnetem Ventil 53, 23, 33, 42, 45.

i) Filterkerze "trocknen"

Die in der Filterkerze und im Filtergehäuse vorhandene benetzende Flüssigkeit wird durch Evakuieren mit der Wasserringpumpe 32 über Ventil 31 entfernt. Zur Beschleunigung der Trocknung wird während der Laufzeit der Wasserringpumpe das Filtergehäuse über Ventil 62 für ca. 3 min mit Druckluft geflutet.

k) Sammelbehälter "entleeren"

Der im Sammelbehälter 43 vorhandene Rest Aethanol wird über Leitung 46 und Ventil 48 entleert.

Diese Vakuumpumpe 32 ist zweckmäßig die Wasserringpumpe, die auch Bestandteil der Gefriertrocknungsanlage 1 ist und z. B. während der Schlußphase des Sterilisationsschrittes zur Entfernung des Heißdampfes eingesetzt werden kann. Mit Hilfe der Wasserringpumpe 32 wird im Filtergehäuse 11 und im Behälter 43 ein ausreichend niedriger Druck erzeugt, so daß alle Alkoholreste entfernt werden. Danach wird das Ventil 23 geschlossen. Die Leitung 22 wird über das Ventil 23 belüftet. Der Belüftung des Behälters 43 dient die Leitung 44 mit dem Ventil 45. Um das Innere des Behälters 43 keimfrei zu halten, erfolgt die Belüftung über einen weiteren Sterilfilter 55.

Für den Fall, daß sich die Filterkerze 12 während des Tests als fehlerfrei erweist, bleibt die sterile Seite des Sterilfilters 7 keimfrei. Der Sterilfilter 7 steht deshalb nach dem Trocknungsschritt zur Belüftung der Kammern 2 und 4 der Gefriertrocknungsanlage 1 zur Verfügung. Nach dem Test sind Manipulationen an dem Sterilfilter 7 nicht erforderlich, so daß die Integrität der Filterkerze 12 während des Belüftungsschrittes sichergestellt ist.

Hat sich die Filterkerze 12 auf Grund des Druckhaltetestes als defekt erwiesen, dann ist sie durch eine neue zu ersetzen. Dazu wird das Gehäuse 11 des Sterilfilters 7 geöffnet und der Kerzenaustausch vorgenommen.

Nach diesem Austausch erfolgt zunächst wieder ein Sterilisationsschritt, indem Heißdampf über die Leitung 18 in das Filtergehäuse 7 und in den Behälter 43 eingelassen wird. Zum Abschluß der Sterilisationsphase erfolgt die Entfernung des Dampfes mit Hilfe der Wasserringpumpe 32.

Nach diesem separat von der Anlage 1 durchgeführten Sterilisationsschritt steht der Filter 7 für die Durchführung eines erneuten Integritätstests zur Verfügung. Die Wahrscheinlichkeit, daß eine neue Filterkerze defekt ist, ist zwar nicht sehr groß; durch den erneuten Test wird jedoch zusätzlich kontrolliert, ob die neue Filterkerze 12 korrekt eingesetzt wurde.

Zweckmäßig ist ein automatischer Ablauf des Inbetriebnahme- und Testverfahrens. Auf die Darstellung einer zentralen, den Ventilen zugeordneten Steuereinrichtung würde jedoch aus Übersichtsgründen verzichtet.

Die Erfindung ist anhand der Gefriertrocknungsanlage 1 beispielhaft erläutert worden. Bei anderen industriellen Anlagen (Autoklaven, Fermentatoren usw.) mit steril zu belüftenden Vakuumkammern ist die Erfindung ebenfalls einsetzbar. Auch kann anstelle des Druckhaltetests ein anderer Integritätstest durchgeführt werden. Wesentlich ist, daß der Filter vor dem Test in eingebautem Zustand sterilisiert wird und der Test so durchgeführt werden kann, daß bei intakter Filterkerze keine Keime auf die sterile Seite des Filters gelangen können.

## Ansprüche

1. Verfahren zum Betrieb einer industriellen Anlage (1) mit mindestens einer Vakuumkammer (2, 4), welche vor der Inbetriebnahme sterilisiert wird und nach Beendigung des in der Vakuumkammer durchgeführten Verfahrens mit sterilem Gas belüftet wird, indem das der Belüftung dienende Gas durch ein Sterilfilter (7) in die Vakuumkammer eingelassen wird, dadurch gekennzeichnet, daß unmittelbar nach der gleichzeitig durchgeführten Sterilisation von Kammer (2, 4) und Filter (7) die Anlage (1) in Betrieb genommen und der in der Kammer durchzuführende Prozeß eingeleitet wird, daß der Filter (7) nach seiner Sterilisation in eingebautem Zustand auf Integrität geprüft wird und daß für den Fall, daß der Filter (7) defekt ist, das Filtermaterial (12) ausgetauscht und der Filter separat von der Vakuumkammer (2, 4) erneut sterilisiert wird.

2. Verfahren nach Anspruch 1, dadurch gekennzeichnet, daß nach einem Austausch der Filterkerze (12) und nach seiner separaten Sterilisation erneut ein Integritätstest durchgeführt wird.

3. Verfahren nach Anspruch 1 oder 2, dadurch gekennzeichnet , daß gleichzeitig mit der Sterilisation von Vakuumkammer (2, 4) und Filter (7) ein Auffangbehälter (43) sterilisiert wird, der an die vom Filter (7) zur Vakuumkammer (2, 4) führende Leitung (6) angeschlossen ist.

4. Verfahren nach einem der vorhergehenden Ansprüche, dadurch gekennzeichnet, daß der Sterilfilter (7) in eingebautem Zustand einem Druckhaltetest unterworfen wird.

5. Anlage zur Durchführung des Verfahrens nach einem der vorhergehenden Ansprüche, dadurch gekennzeichnet, daß der Sterilfilter (7) zwischen Anlage (1) und Belüftungsgasquelle (5) installiert ist und daß der Anschluß (8) des Sterilfilters (7), der mit der Belüftungsgasquelle in Verbindung steht, auch mit einer Heißdampfquelle (17) in Verbindung steht.

6. Anlage nach Anspruch 5, dadurch gekennzeichnet, daß an die vom Sterilfilter (7) zur Anlage (1) führende Leitung (6) ein Behälter (43) über die Leitung (41) mit dem Ventil (42) angeschlossen ist.

7. Anlage nach Anspruch 5 oder 6, dadurch gekennzeichnet, daß der Sterilfilter (7) ein Filtergehäuse (11) mit insgesamt vier Anschlüssen (8, 9, 21, 22) aufweist, von denen drei (8, 21, 22) in das Innere des Filtergehäuses (11) münden, während der vierte (9) mit dem Innenraum einer zylindrischen Filterkerze (12) verbunden ist.

8. Anlage nach Anspruch 7, dadurch gekennzeichnet, daß einer (8) der Anschlüsse (8, 21, 22) im unteren Bereich des Filtergehäuses (11) angeordnet ist und wahlweise mit einem Behälter mit Benetzungsflüssigkeit und einem Behälter mit Alkohol verbindbar ist.

9. Anlage nach Anspruch 8, dadurch gekennzeichnet, daß einer (22) der Anschlüsse (8, 21, 22) an den unteren Bereich des Filtergehäuses (11) angeordnet ist und der Abführung von Flüssigkeiten und/oder Kondensat dient.

10. Anlage nach einem der Ansprüche 7 bis 9, dadurch gekennzeichnet , daß der Anschluß (9) mit einer Wasserringpumpe verbindbar ist.

Europäisches
Patentamt

EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 739 169 (SMEJA)<br>* Seite 5, Absatz 2 *<br>--- | | A 61 L 2/06<br>F 26 B 5/06 |
| A | FR-A-2 353 300 (LAGUILHARRE)<br>* Seite 10, Absatz 3 *<br>--- | | |
| A | DE-A-3 708 734 (SATORIUS)<br>* Spalte 3, Zeilen 5-13 *<br>----- | | |

RECHERCHIERTE SACHGEBIETE (Int. Cl.4)

A 61 L
F 26 B

Der vorliegende Recherchenbericht wurde für alle Patentansprüche erstellt

| Recherchenort | Abschlußdatum der Recherche | Prüfer |
|---|---|---|
| DEN HAAG | 09-03-1989 | COUSINS-VAN STEEN G.I.L. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)